# EUROPÄISCHE PATENTSCHRIFT

(11) **EP 2 942 035 B1**
(45) Veröffentlichungstag und Bekanntmachung des Hinweises auf die Patenterteilung: **30.05.2018**
(21) Anmeldenummer: 15001403.3
(22) Anmeldetag: 05.10.2011
(51) Int. Cl.: A61L 27/04, A61L 27/14, A61F 2/30, A61F 2/44, A61F 2/48

(54) **MEDIZINISCHES IMPLANTAT FÜR DEN ZWISCHENWIRBELRAUM**
MEDICAL IMPLANT FOR THE INTERVERTEBRAL SPACE
IMPLANT MÉDICAL POUR L'ESPACE INTERVERTÉBRAL

(30) Priorität: 05.10.2010 DE 102010041959
(43) Veröffentlichungstag der Anmeldung: 11.11.2015
(62) Teilanmeldung aus: 11801572.6
(73) Patentinhaber: Aces Ingenieurgesellschaft mbH, 70794 Filderstadt (DE)
(72) Erfinder: TRAUTWEIN, Frank, 70794 Filderstadt (DE); HEUER, Frank, 70794 Filderstadt (DE); FRANKE, Jörg, 39118 Magdeburg (DE); KOTHE, Ralph, 22397 Hamburg (DE); LILJENQVIST, Ulf, 48147 Münster (DE); MATGÈ, Guy, 8229 Mamer (LU); PUTZIER, Michael, 14532 Stahnsdorf (DE)
(74) Vertreter: Patentanwälte Magenbauer & Kollegen Partnerschaft mbB

(56) Entgegenhaltungen:
- DE-U1- 20 314 708
- US-A1- 2005 112 397
- US-A1- 2006 224 241
- US-B1- 6 200 347

## Beschreibung

Die Erfindung betrifft ein medizinisches Implantat, welches in den Zwischenraum zweier Wirbel eingeführt werden kann um die Wirbelsäule zu stabilisieren. Das erfindungsgemäße Implantat umfasst eine Vielzahl dünner Platten, wodurch vorteilhafte Eigenschaften bezüglich der Herstellung, Implantation und der Funktion im Körper realisiert werden können.

### Stand der Technik

Bei Erkrankungen der Wirbelsäule ist es oft erforderlich, eines oder mehrere Segmente zu stabilisieren. Hierfür gibt es eine Vielzahl von Zwischenwirbelimplantaten (auch "Cages" genannt) die eine knöcherne Fusion eines Wirbelsäulenabschnittes zum Ziel haben. Die Implantation solcher Cages kann zahlreiche Komplikationen nach sich ziehen. Beispielsweise können die Implantate aufgrund einer punktuellen Belastung oder kleinen Auflagefläche in die Deckplatte einbrechen, die erwünschte, knöcherne Fusion kann durch "Stress Shielding" ausbleiben, die Implantate können verrutschen oder die Stellung der Wirbelkörper zueinander kann nicht ausreichend korrigiert werden. Außerdem wird in der Regel eine sekundäre Abstützung (z.B. über ein Pedikelschrauben-Stab-System) benötigt, um das Segment soweit zu stabilisieren, dass eine Migration diese Implantate zuverlässig verhindert wird.

Aus WO9501763A1 1993 ist ein Wirbelsäulenimplantat bekannt, welches aus einem korbähnlichen Ring besteht und eine zentrale Öffnung für die Aufnahme von Knochenmaterial besitzt. In (EP1800627A2 2005) wurde beschrieben, dass ein Wirbelsäulenimplantat zur Kontaktflächenvergrößerung aus zwei oder mehreren Ringen bestehen kann, um so ein Einbrechen in die Wirbeldeckplatten zu vermindern. Aus WO0234171A2 2000 sind deformierbare Polymerstreifen bekannt, die in den Wirbelkörperzwischenraum eingebracht werden und zur Lastrichtung ausgerichtet werden können.

Aus den Anmeldungen US6767738B1 2004 und WO2008079953A2 2006 wird jeweils eine Anordnung von zwei oder drei rigide verbundenen Platten vorgeschlagen, die durch den Abstand der Platten einen Raum zur Einbringung von Knochen, Knochenersatzmaterial oder BMP erlauben. Die Platten erfüllen hier die Funktion einer Aufnahme um das mechanisch instabile Knochenmaterial in den Zwischenwirbelraum einzubringen und eine unerwünschte Verteilung im Bandscheibenraum bzw. im Körper zu verhindern. Aufgrund der gewünschten Funktion und der geringen Anzahl der Platten sind keine praktikablen Vorkehrungen vorgesehen die die Abstände der Zwischenräume einer größeren Anzahl von Platten bestimmt. Von Nachteil ist außerdem die erforderliche Gewinnung von Knochen zur Füllung des Zwischenraums, bzw. die Bereitstellung und Einbringung anderer Knochenersatzmaterialien.

Durch WO0045747A1 1998; WO0040177A1 1999 sind aus Knochenmaterial bestehende, geschichtete Anordnungen bekannt.

Aus der US 5,732,469 A ist ein Prothesensystem bekannt, bei dem zumindest Teilbereiche der Prothese aus aufeinander geschichteten, mit Durchbrüchen versehenen Materiallagen hergestellt sind, wobei die Dicke der Materiallagen 150 Mikrometer oder weniger angegeben ist und die einzelnen Materiallagen stoffschlüssig miteinander verbunden werden.

Die US 2005/112397 A1 offenbart ein Implantat, das als Schichtaufbau aus durchbrochenen Einzellagen aufgebaut ist und bei dem die Einzellagen mechanisch und/oder physikalisch, insbesondere stoffschlüssig durch Weich- oder Hartlöten, verbunden sind.

Der US 5,108,432 A sind ein künstliches Hüftgelenk und eine Schienbeinprothese zu entnehmen, die beide mit durchbrochenen Platten ausgerüstet werden können. Die Platten sind hierbei mit Durchbrüchen und Schlitzen versehen.

Aus der WO 2006/091097 A2) ist ein Schichtaufbau aus Einzellagen eines porösen Materials, insbesondere aus Metallfolien, bekannt, der zum Ersatz von menschlichen oder tierischen Knochen dienen soll, wobei die Einzellagen parallel zueinander angeordnet und direkt aufeinander geschichtet sind.

Die zuvor genannten Erfindungen bestehen aus oder erlauben die Einbringung von Knochen oder Knochenersatzmaterial in den Zwischenwirbelraum, ermöglichen jedoch weder die Korrektur der Wirbelstellung (Winkel und Versatz) noch bieten sie Lösungen zur gleichmäßigen Verteilung der Belastung auf die Deckplatten der Wirbelkörper. Aufgrund der nicht einstellbaren Höhe der Implantate sind während der Implantation teilweise hohe Einschlagkräfte und eine (Über)Distraktion der Endplatten erforderlich.

Aus WO0044319A1 1999 ist ein expandierbares Wirbelsäulenimplantat bekannt. Durch einen kleinen Bandscheibenzugang können zahlreiche zusammengefaltete Elemente eingebracht und durch eine Komprimierung so aufgestellt werden, dass sie die Wirbel distrahieren.

Aus DE 19816832 C1 und aus US 2010/010633 A1 sind Wirbelsäulenimplantate bekannt, welche mindestens eine Welle mit exzentrisch daran befestigten Scheiben besitzen. Die Welle ist verdrehbar, so dass die Scheiben herausgestellt werden und das Implantat an Bauhöhe gewinnt. Ein potentieller Nachteil dieser Ausgestaltung liegt in der Notwendigkeit einer steifen Verbindung zwischen der Welle und den Scheiben. Des Weiteren sind diese Wirbelsäulenimplantate auf die Distraktion beschränkt, wobei ein Verschieben der Wirbel zueinander aufgrund der drehbaren Welle und der entstehenden Reibung zwischen den Scheiben und den Deckplatten nicht vollständig ausgeschlossen werden kann. Die klinische Erfahrung zeigt, dass durch den weitgehend zylindrischen Querschnitt eine Vielzahl dieser Implantate in die Deckplatte einbrechen und diese teilweise schwer zu revidieren sind.

Aus US 2010/016968 A1 und WO 2007/048012 A2 sind Methoden und Implantate bekannt mit denen es möglich ist, eine Reposition eines abgeglittenen Wirbels durchzuführen. Dabei werden zwei implantierte Hälften gegeneinander verschoben bis die Endstellung erreicht ist. Hier basieren die Ansätze auf temporär implantierte Apparaturen die nach der finalen Versorgung wieder entfernt werden müssen.

Aus der US 2006/224241 A1 ist eine verstellbare Wirbelimplantateinrichtung zum Einsetzen in einen Wirbelsäulenraum vorgesehen, bei der eine Expansion durch eine Relativbewegung zwischen wenigstens zwei Gruppen von axial gestapelten Segmente vorgesehen sind, die auf einer zentralen Bereitstellungsstange angeordnet sind. Jedes Segment enthält eine zentrale Platte, an der Platten oder Blattstrukturen schwenkbar angebracht sind, um eine Verschwenkung aus einer zusammengeklappten, nicht aufgeweiteten Position in eine offene, expandierte Position zu ermöglichen.

Die US 6,200,347 B1 offenbart ein Knochen-Verbundtransplantat, das für die Implantation in einen Patienten eingerichtet ist. Das Knochen-Verbundtransplantat umfasst zwei oder mehrere verbundene, diskret ausgebildete Knochenanlageplatten sowie einen oder mehrere biokompatible Verbinder, die zwischen den Knochenanlageplatten angeordnet sind und zusammen mit diesen das Verbundknochentransplantat bilden. Das Verbundknochentransplantat ist derart ausgebildet, dass es sich nach der Implantation nicht verschiebt, dehnt oder dreht.

Aus der DE 20314708 U1 ist ein Wirbelsäulenimplantat zur Überbrückung des Zwischenraumes zwischen den Endflächen von zwei Wirbelkörpern mit veränderbarer Höhe bekannt. Das Wirbelsäulenimplantat weist mehrere nebeneinander angeordnete Distanzelemente auf, die in einer Niedrigstellung schräg zu einer Horizontalebene verlaufen, die im wesentlichen parallel zu Endflächen der Wirbelkörper angeordnet ist, und die in einer Hochstellung gegenüber der Niedrigstellung aufgerichtet sind und zumindest annähernd senkrecht zu der Horizontalebene verlaufen.

Neben den verschiedenen vorhergehend beschriebenen Unzulänglichkeiten beinhalten alle bisher bekannten Implantate keine oder lediglich einfache Verzahnungen an den Knochen-Kontaktflächen zur Erhöhung des Widerstandes gegen Migration. Die mechanische Erzeugung definierter Zahngeometrien mit einem Hinterschnitt ist aufgrund der bisher verwendeten Bauweisen und Fertigungsverfahren nicht bekannt. Bei der Verwendung von Knochen als Implantatwerkstoff verbietet die geringe Festigkeit des Werkstoffs die Ausgestaltung und Anwendung derartiger Zahngeometrien.

### Darstellung der Erfindung

### Technische Aufgabe

Die Aufgabe der vorliegenden Erfindung besteht in der Bereitstellung eines Implantats zur Stabilisierung der Wirbelsäule über den Zwischenwirbelraum. Wichtige Aufgaben des erfindungsgemäßen Implantats sind eine gleichmäßige Verteilung der Belastung über einen möglichst großen Teil der Kontaktfläche sowie die einfache aber sichere Verankerung in den Kontaktflächen ohne diese mechanisch zu schwächen. Dabei kann eine Anpassung von Höhe und Winkel des Implantats im Situs sowie die Korrektur von translatorischen Verschiebungen vorgenommen werden. Vorzugsweise entfällt damit die Notwendigkeit zur Befüllung des Implantats mit Knochen oder Knochenersatzmaterialien. Das Erfindungsgemäße Implantat sollte außerdem kostengünstig in der Herstellung sein und die intraoperative Handhabung vereinfachen.

Die Aufgabe wird für ein medizinisches Implantat zur Platzierung zwischen Knochen, insbesondere zwischen Wirbelkörper, mit den Merkmalen der Ansprüche 1 und 14 gelöst. Vorteilhafte Ausgestaltungen dieser medizinischen Implantate gehen aus den Unteransprüchen hervor. Die Platten können knochenseitig eine filigrane Struktur besitzen, wobei durch Hinterschnitte in der Verzahnungsstruktur eine nochmalige Steigerung der Verankerungsfestigkeit in den Endplatten ermöglicht werden kann. Durch das gezielte Einbringen von weiteren Konturen bzw. Strukturen an und/oder in den Platten können unterschiedliche, optionale Funktionen des Implantats erzeugt und miteinander kombiniert werden. Es können zum Beispiel bestimmte Bereiche der Platten einen elastischen Charakter aufweisen und sich damit der Endplattenkontur anpassen bzw. einem "Stress-Shielding" entgegenwirken. Die Steifigkeit kann außerdem so eingestellt werden dass sie der von Knochen entspricht und dadurch einen Dehnungsreiz an das umgebende Gewebe gesendet wird. Im Verbund können die Platten mit einem Aktuator, dessen Verstellmechanismus, z.B. mit einer exzentrischen Welle, einem mechanischen, elektrischen oder auf formgedächtniseigenschaften basierten Mechanismus realisiert werden kann, so verbunden werden, dass sie Relativbewegungen vollziehen können, um beispielweise das Bandscheibenfach zu distrahieren oder zwei Wirbel relativ zueinander zu reponieren.

Erst die Verwendung von parallel angeordneten Platten dünner Wandstärke erlaubt die kostengünstige Fertigung der notwendigen, teilweise komplexen Strukturen. Die Konturen können hierzu bevorzugt durch ein photo-chemisches Ätzverfahren hergestellt oder durch energiereiche Strahlung (z.B. Laserstrahlen, Elektronenstrahlen) bzw. durch einen Wasserstrahl aus den Platten geschnitten werden. Darüber hinaus sind die Konturen ganz oder teilweise durch Drahterodieren oder Spritzgießen (aus Metall- oder Kunststoff) herstellbar.

Vorteilhaft bei dem erfindungsgemäßen Implantat ist der durch den besonderen Verzahnungsaufbau mögliche sehr hohe Widerstand gegen Migration und Einbrechen in die Grund- und Deckplatten. Dadurch ergibt sich eine sehr hohe Primärstabilität, wodurch eine zusätzliche Stabilisierung über ventrale Platten oder dorsale Stabsysteme bei einem Teil der Patienten überflüssig wird. Durch die Vielzahl der sich ergebenden Kanäle bzw. Gitterstrukturen und einer elastischen Lagerung der Verzahnung bzw. Einstellung der Implantatsteifigkeit über geeignete Aussparungen wird außerdem ein großer Stimulus zur Bildung von Knochen erzeugt ohne dass das Implantat mit zusätzlichem Knochen oder Knochenersatzmaterial befüllt werden muss. Durch die verschiedenen Verstellmöglichkeiten wird einerseits eine optimale Anpassung an die Patientenanatomie erreicht, andererseits wird die Implantation bei den Ausführungen vereinfacht die nach der Einbringung distrahiert oder im Winkel eingestellt werden können. Eine weitere Variante stellt zudem eine Möglichkeit der einfachen Korrektur der AP-Ausrichtung der Wirbelkörper über eine im Implantat integrierte Funktionalität vor.

### Kurze Beschreibung der Zeichnungen

Fig. 1 zeigt die implantierbare Erfindung zur Fixierung eines Wirbels.

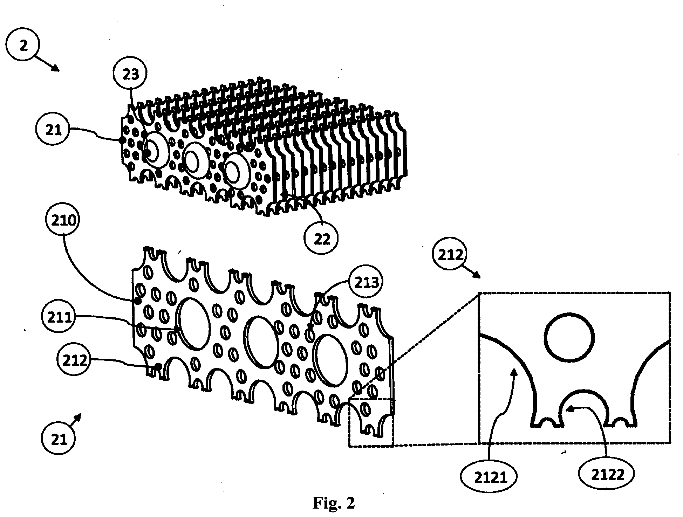

Fig. 2 beschreibt eine nicht zur Erfindung gehörende Ausführungsvariante,
Fig. 3 beschreibt eine nicht zur Erfindung gehörende Ausführungsvariante,
Fig. 4 zeigt eine nicht zur Erfindung gehörende Implementierung von Verankerungselementen zur gleichmäßigen Lastverteilung der Platten,
Fig. 5 illustriert den Aufbau eines erfindungsgemäßen distrahierbaren Implantats,
Fig. 6 zeigt einen Implantataufbau für eine Modifikation des in Fig. 5 gezeigten Implantats,
Fig. 7 beschreibt den Aufbau einer weiteren erfindungsgemäßen Ausgestaltungsvariante,
Fig. 8 zeigt ein Implantat für den postero-lateralen Zugang und die Realisierung einer ungleichmäßigen Höhenzunahme zur über die Implantatbreite,
Fig. 9 und 10 zeigen zwei beispielhafte Methoden, wie die Erfindung implantiert werden kann,
Fig. 11 zeigt wie ein erfindungsgemäßes Wirbelsäulenimplantat aufgebaut ist, um einen Wirbel zu reponieren,

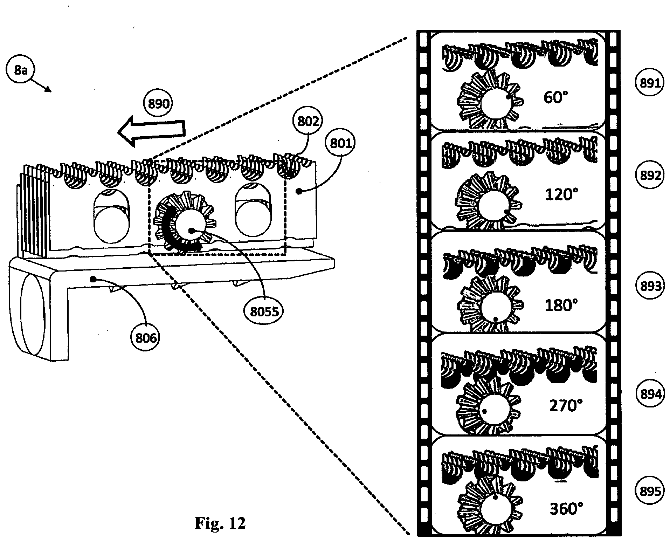

Fig. 12 erläutert die mechanische Umsetzung der Korrekturbewegung des in Fig. 11 gezeigten, erfindungsgemäßen Implantats,
Fig. 13 illustriert die Arbeitsschritte, die bei der Implantation der zuvor genannten Ausgestaltungsform zu berücksichtigen sind,
Fig. 14 beschreibt eine erfindungsgemäße alternative Ausführungsform zu der in Fig. 12 gezeigten Ausgestaltungsvariante.
Fig. 15 zeigt den Implantiervorgang für die in Figur 14 dargestellte Variante.

Die technischen Lösungen sind nachfolgend oft beispielhaft beschrieben. Dies soll als Mittel zur Erläuterung des zugrundeliegenden Gedankens aufgefasst und nicht als auf die jeweilige konkrete Darstellung beschränkt verstanden werden. Die hier im Einzelnen beschriebenen Ausführungsformen bilden eine kombinatorische Grundlage der Erfindung, das heißt die Ausführungsformen können einzeln oder ein Kombination miteinander ausgeführt werden.

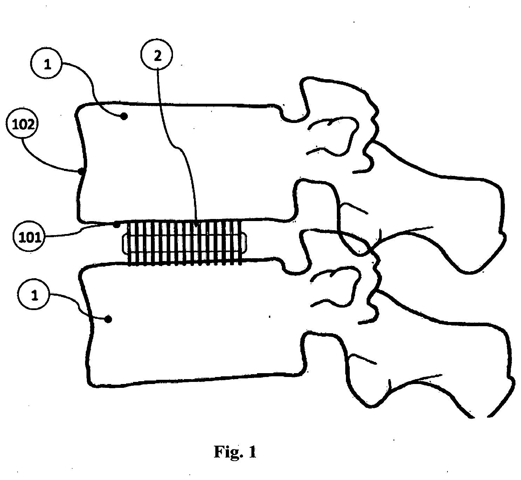

Das in der Figur 1 schematisch dargestellte erfindungsgemäße Implantat 2 wird in den Zwischenraum zweier Wirbel 1 eingeführt, um die Wirbelsäule zu stabilisieren. Dabei entsteht mindestens eine Kontaktfläche zwischen Implantat und den Deckplatten 101 der angrenzenden Wirbel 1. Das Wirbelsäulenimplantat 2 besteht aus einer Vielzahl von Platten, die zusammen mit ihren Zwischenräumen mit Hilfe mindestens eines Verbindungselements miteinander verbunden sind. Die Platten weisen eine Wandstärke von 0,05 bis, 3mm, insbesondere zwischen 0,2 bis 1,5 mm auf. Die Breite der Zwischenräume orientiert sich an der Größe der Trabekelstruktur des Knochens und beträgt ebenfalls zwischen 0,05 und 3mm, insbesondere zwischen 0,2 und 1,5mm.

Die Platten können beispielsweise durch Spritzgießen aus Metall (Metal Injection Molding), Kunststoff oder aus anderen biokompatiblen bzw. bio-resorbierbaren Werkstoffen, oder einer Kombination dieser Werkstoffe hergestellt werden. Andere besonders geeignete Fertigungsverfahren für die Herstellung der Platten sind das Ausschneiden mit Hilfe energiereicher Strahlen (Elektronenstrahl, LASER, Wasserstrahl) oder das Herausätzen über ein photochemisches Ätzverfahren aus Metallbändern, Blechen oder Platten (Photo-Etching). Die genannten Herstellungsverfahren bieten eine Vielzahl von Ausgestaltungsmöglichkeiten der Platten und erlauben die kostengünstige Integration von Funktionen in das Implantat, die durch die bekannten ein- oder zweiteiligen, im Wesentlichen monolithischen Ausführungsformen nicht realisiert werden können. So ist es zum Beispiel auf einfache Weise möglich, Strukturen, Profile, Führungen, Öffnungen oder Verzahnungen 212 mit unterschiedlicher Austragungstiefe in die Platten zu integrieren, Verzahnungen mit Hinterschnitt herzustellen oder Zähne jeweils versetzt zur nächsten Platte anzuordnen. So kann die Knochen-Auflagefläche z.B. durch eine Vielzahl kleiner Verzweigungen 2121 eine "Mikropenetration" der Deckplatte 101 ermöglichen, wobei die Verzweigungen einen Hinterschnitt aufweisen können und dadurch bereits nach einer kurzen Einheilungsphase eine primärstabile Verankerung im Knochen ermöglichen und die Verankerungsfestigkeit insbesondere unter Zugbelastung deutlich erhöht wird. Durch den Plattenaufbau und die damit möglichen Fertigungsverfahren können semi-permeable Poren, Kapillaren oder Öffnungen hergestellt werden, welche den Aufbau eines osmotischen Drucks im Inneren des Implantats hervorrufen, und dieser wiederum zur Stoffwechselförderung und Zelldifferenzierung genutzt werden kann. Um die Plattenoberfläche osteokonduktiv oder/und osteoinduktiv zu gestalten, können die Platten mit einem geeigneten Material beschichtet sein oder in einer definierten Rauigkeit hergestellt sein. Außerdem kann der Werkstoff aller oder einzelner Platten so gewählt werden, dass diese über die Zeit resorbierbar sind. Zur definierten Resorption über einen langen Zeitraum bietet es sich an, die Platten mit unterschiedlicher Dicke bzw. aus unterschiedlichen Materialien mit unterschiedlicher Resorptionsrate herzustellen.

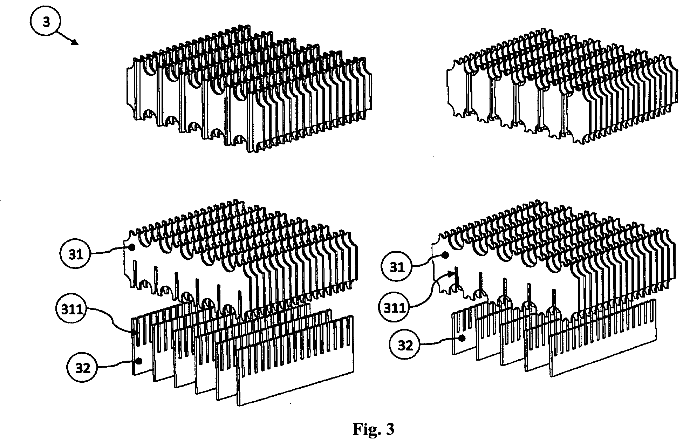

Bei einer nicht zur Erfindung gehörenden Ausführungsvariante 3 gemäß der Figur 3 können die Platten so ausgestaltet sein, dass sie ineinander steckbar sind Fig. 3. Dies kann dadurch erreicht werden, indem die Platten 31 und/oder 32 schlitzförmige Verbindungsöffnungen 311 aufweisen, die jeweils miteinander kompatibel sind und eine formschlüssige Verbindung ermöglichen. Der Abstand der Schlitze 311 in der Verbindungsplatte 32 definiert dabei die Teilung und damit den Abstand des Zwischenraumes 22.

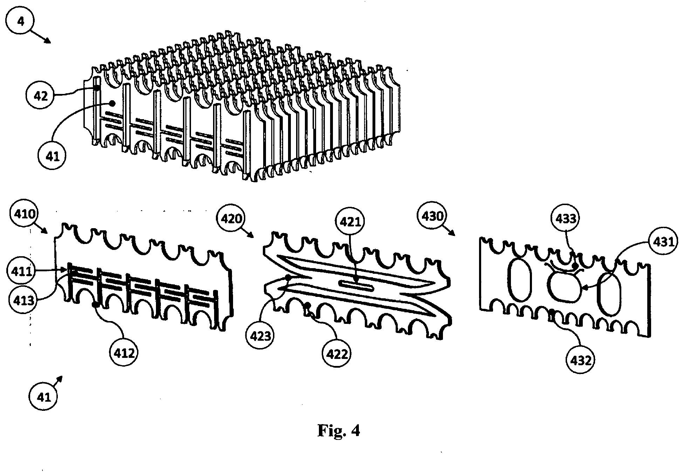

Bei einer ebenfalls nicht zur Erfindung gehörenden Ausgestaltungsform gemäß der Figur 4 ist beispielhaft die dynamische Ausgestaltung der Platten 41 gezeigt. Damit ist das Implantat in der Lage, sich der Deckplattenkontur 101 anzupassen und damit eine gleichmäßige Kraftverteilung auf die Deckplatte zu ermöglichen, ohne einen Deckplatteneinbruch infolge lokaler Kraft- und Spannungskonzentrationen zu provozieren. Die Platten 410 können dabei so gestaltet sein, dass jede einzelne die Knochen-Kontaktfläche bildende Haken- oder Zahnstruktur 412 beispielsweise durch einen mäanderförmigen Konturausschnitt 413 federnd gelagert ist.

Ferner wird in der Fig. 4 420 eine federnde Lagerung der Knochen-Kontaktfläche über mehr als einem Hakenelement 422 ermöglicht, indem ein Konturausschnitt 423 größer ausgestaltet ist. Des Weiteren kann eine federnd gelagerte Verbindung der Platten 430 dazu verwendet werden, eine Elastizität oder Steifigkeitsreduktion des gesamten Implantats zu erzielen. Dies kann erreicht werden indem die Verbindungsöffnungen 431 einen benachbarten Konturausschnitt 433 erhalten, welcher eine elastische Nachgiebigkeit im Bereich der Lastübertragungsfläche der Verbindungs- bzw. Einstellöffnung 431 ermöglichen.

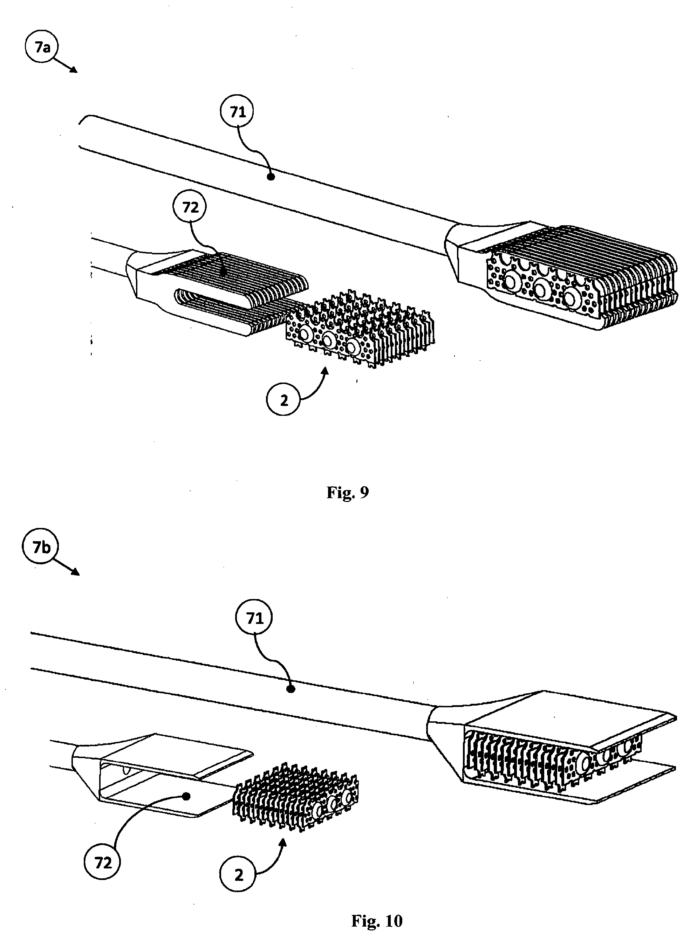

Fig. 4 zeigt außerdem verschiedene Ausschnittformen 411, 421, 431 zur Aufnahme von weitgehend rechtwinklig zu den Platten 41 angeordneten Verbindungselementen 42, 23, 5051, 5052, 5053 usw.. Die Verbindungselemente können als Schraube, Stift, Rohr oder Achse, mit rundem, ovalem, rechteckigem oder Polygon-Querschnitt ausgeführt sein. Verbindungselemente in Plattenform 32, 42 eignen sich zur gezielten Einstellung eines Abstands des Zwischenraumes 22 zwischen den Platten. Der Abstand der Platten ist dabei bevorzugt so zu wählen, dass er mit der Strukturgröße der Knochentrabekel kompatibel ist, also von ca. 0,02 bis 3mm und insbesondere zwischen 0,2 und 1,5mm. Eine alternative Möglichkeit zur Einstellung des Zwischenraumabstands 22 besteht in der Abstufung des Querschnitts von Verbindungselement 42, 5051, 5052, 5053 usw. entlang seiner Achse und der Verbindungsöffnung 421, 431 der Platten 41. Bei einer großen Plattenanzahl ist jedoch insbesondere die Verwendung von Scheiben 5020, Fig. 10, Ringen oder ähnlichen Elementen mit einer Dicke die dem gewünschten Zwischenraumabstand 22 entspricht vorteilhaft. Hierzu werden die Abstandsscheiben 5020 jeweils zwischen den Platten 41 auf den Verbindungselementen 23, 5051, 5052, 5053 usw. angeordnet. Schließlich ist eine zwischen den Platten 41 räumlich versetzte Prägung der selbigen dazu geeignet, einen definierten Abstand 22 zwischen den Platten einzustellen.

Das Implantat kann derart gestaltet sein, dass die Montage von Platten 21, Verbindungselement 23 bzw. 32 und ggf. separaten Abstandsscheiben- oder Ringen 5020 patientenspezifisch während der Operation erfolgt. Dies kann dadurch erzielt werden, indem ein Instrument in das Bandscheibenfach eingebracht wird, welches einen einstellbaren oder deformierbaren Bereich besitzt mit dem sich die Deckplattenkontur erfassen oder temporär abformen lässt. Das Instrument wird wieder aus dem Bandscheibenfach entfernt und das Wirbelsäulenimplantat kann während der Operation aus einzelnen Platten 21 unterschiedlicher Höhe und Breite der Deckplattenkontur entsprechend zusammengebaut werden.

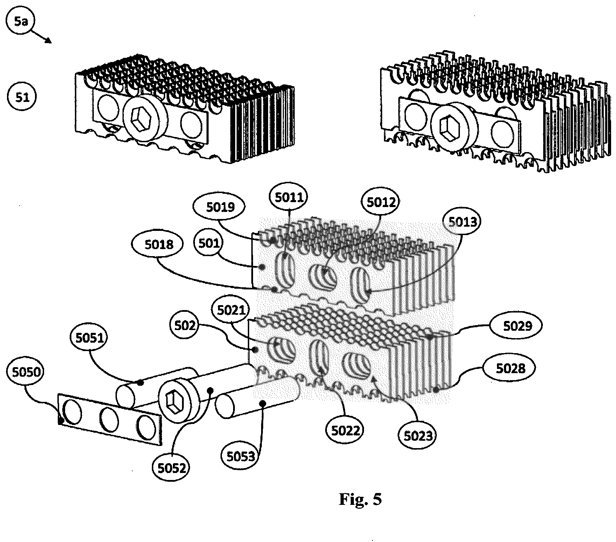

In der Figur 5 wird eine erfindungsgemäße Ausführungsvariante eines Implantats 5a mit verstellbarer Höhe vorgestellt. Beim Einführen des Wirbelsäulenimplantats 5a in den Zwischenwirbelraum weist der Plattenverbund, bestehend aus oberen 501 und unteren 502 Platten zunächst eine minimale Höhe auf, wobei die Verzahnungsstrukturen 5019 und 5028 durch abgeflachte Profile 5018 und 5029 überdeckt sind und damit das Einführen des Implantats erleichtern. Erst nach der Höhenverstellung, bei der die Plattenverbünde 501 und 502 gegenseitig verschoben werden, ragen die Verzahnungsstrukturen 5019 und 5028 über die abgeflachten Profile 5018, 5029 hinaus in die Deckplatten 101 und dienen dort zur Verankerung. Die Platten 501 und 502 sind so gestaltet, dass sie diverse Verbindungsöffnungen besitzen. Dabei wird zwischen den Öffnungen für die Lagerung 5011, 5013, 5021 und 5023 und den Öffnungen für die Einleitung der Relativbewegung 5012 und 5022 der Platten unterschieden. Die Orientierung der Öffnungen 5011-5013 ist dabei zur Erzielung einer Relativbewegung zwischen den Plattenverbünden 501 und 502 von der Orientierung der Öffnungen 5021-5023 verschieden, in einer bevorzugten Ausführung um 90° gedreht. Gelagert werden die Platten 501 und 502 in diesem Ausführungsbeispiel über zwei Achsen 5051 und 5053. Die Relativbewegung der Platten erfolgt über einen Aktuator, welcher in diesem Beispiel über eine Exzenterwelle 5052 und ein damit verbundenes Instrument zur Übertragung einer Drehbewegung realisiert ist. Durch Drehung der Exzenterwelle 5052 bewegt sich der Ausschnitt 5012 und damit der Plattenverbund 501 analog zur Stellung des Kurbelradius der Exzenterwelle 5052. Eine senkrecht zur Welle angeordnete Platte 5050 oder ein das Implantat umschließender Rahmen hält dabei die Verbindungselemente 5051, 5053, die Exzenterwelle 5052 und indirekt über die Lager 5051, 5053 den unteren Plattenverbund 502 in einer konstanten Position. In den weiteren Ausführungen wird der Rahmen 5050 nicht weiter dargestellt um einen Einblick auf den inneren Aufbau zu gestatten.

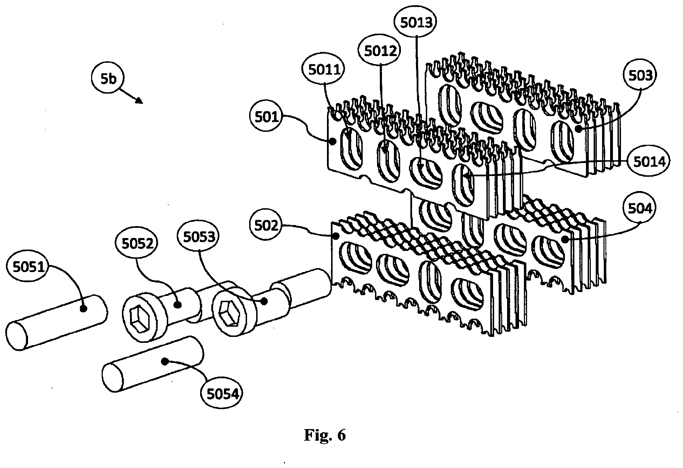

Nachteilig bei der in Fig. 5 dargestellten Variante 5a ist die Kippneigung des oberen Plattenverbunds 501 über die Exzenterwelle 5052. Die Ausgestaltungsform 5a kann deshalb gemäß der Figur 6 dahingehend verbessert werden, dass zwei Exzenterwellen 5052 verwendet werden, wie dies bei der Variante 5b der Fall ist. Dies verhindert die Kippneigung der Plattenverbünde und ermöglicht die separate Höheneinstellung. Durch Gestaltung der die Relativbewegung beeinflussenden Ausschnitte 5012 bzw. die Position und Länge des Abschnitts der Exzentrizität auf der Exzenterwelle 5052 ist es möglich, nur Teile des jeweiligen Plattenverbunds zu verstellen. So ist es beispielsweise möglich, beide Bereiche 52 oder nur den hinteren Bereich oder nur den vorderen Bereich bei Verdrehung der Exzenterwellen 5052, 5053 in der Verstellhöhe zu variieren.

Gemäß der Fig. 6 sind hierzu vier verschiedene Plattenverbünde 501, 502, 503 und 504 vorgesehen, die sich in ihren Verbindungsöffnungen z.B. 5011, 5012, 5013 und 5014 unterscheiden. Der Unterschied kann beispielsweise durch eine alternative Orientierung der Verbindungsöffnungen oder deren Abmessungen erreicht werden. Außerdem sind die Exzenterwellen mit je zwei Abschnitten dargestellt die einen unterschiedlichen Kurbelradius aufweisen.

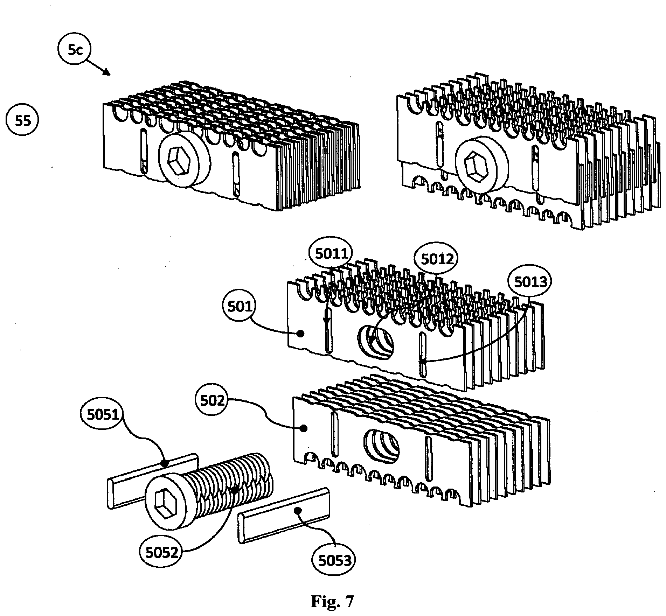

Die Ausgestaltung der Höhenverstellung kann außerdem so gestaltet sein, dass die Platten 501 und 502 bezüglich der Ausschnitte identisch sind und die Exzenterwelle 5052 eine Vielzahl von Einzelexzentern mit gleichem oder unterschiedlichem Kurbelradius besitzt, wie dies bei der Ausführungsform 5c gemäß der Figur 7 dargestellt ist. Damit ist es möglich entlang der Exzenterwelle 5052 unterschiedliche Relativbewegungen zu erzielen. Zur Verringerung der vorher erwähnten Kippneigung bei nur einer Exzenterwelle können außerdem überwiegend rechteckige Verbindungselemente 5051 und 5053 verwendet werden.

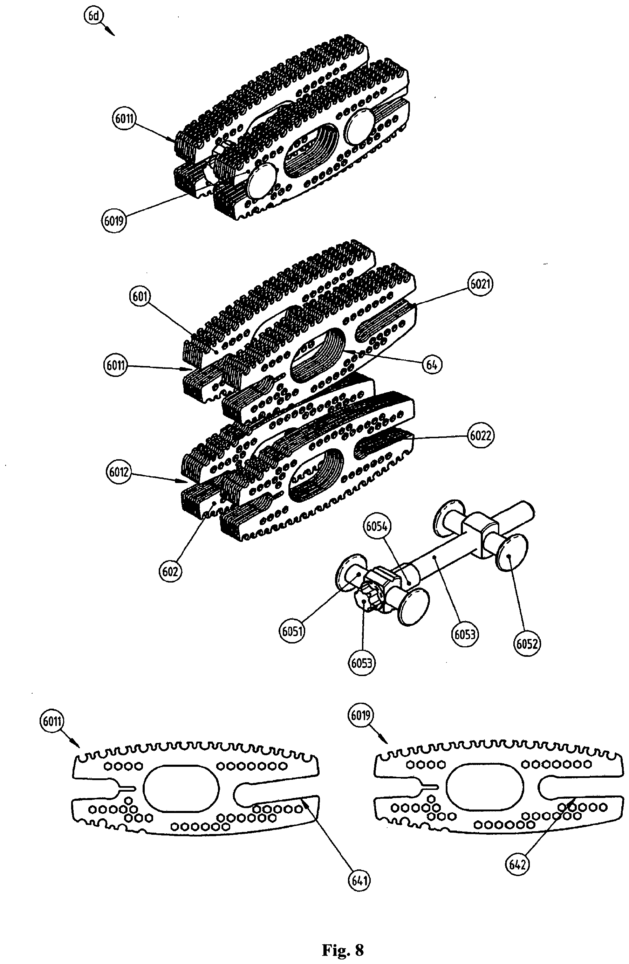

Eine für den postero-lateralen Zugang optimierte Ausgestaltungsform 6d ist in der Fig. 8 dargestellt. Das hier gezeigte Implantat 6d kann einen oder mehrere Ausschnitte 64 für die Anlagerung von Knochenmaterial besitzen. Zur besseren Distraktion des Bandscheibenfaches kann der anteriore Aufrichtwinkel des Implantats der vorderen Führungsnuten 6012, 6022 der Platten 601, 602 von medial nach lateral angepasst werden. Dabei wird der Winkel der vorderen Nut 6021, 6022 bei jeder Platte geändert, z.B. von lateral 6011 nach medial 6019, so dass die Höhenzunahme ein schräg verlaufendes Profil aufweist und damit optimal an die Geometrie der Deckplatten angepasst ist.

Für die Implantation des erfindungsgemäßen Implantats 2 werden in den Figuren 9 und 10 zwei Implantationsinstrumente 7a und 7b beispielhaft gezeigt. Zum Einen kann das Instrument 7a so ausgestaltet sein, dass es aus einem Schaft 71 mit einem distalen Ende 72 besteht. Die Platten des Implantats werden vom distalen Ende 72 formschlüssig umgeben, so dass die Platten mit den Hakenelementen durch das Instrument vor einem unbeabsichtigten Verbiegen beim Einführen geschützt sind, ohne die Höhe des Implantats durch die Wandstärke des Instruments zu vergrößern. In der Ausführung 7b wird ein Instrument für ein anders orientiert einzubringendes Implantat gezeigt welches die gleichen präventiven Funktionen der Ausführung 7a erfüllt, jedoch die Gesamthöhe während der Implantation geringfügig vergrößert. Durch den Schaft 71 kann das Implantat mit dem Instrument z.B. über eine durchgehende Gewindestange verbunden werden, so dass das Implantat sicher gehalten wird und ggf. auch explantiert werden kann.

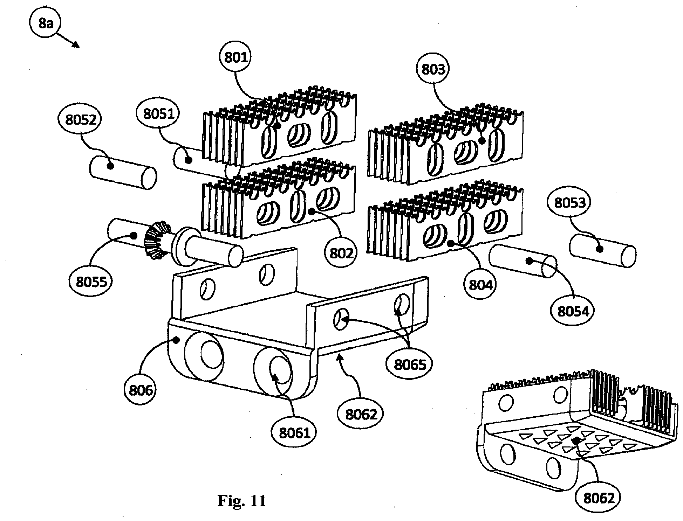

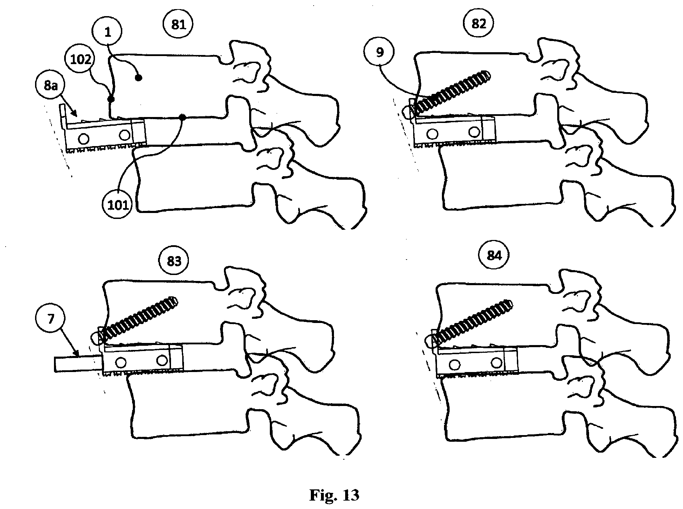

In Zusammenhang mit der Figur 11 wird die Realisierung eines Repositionsmechanismus in Form eines Wirbelsäulenimplantats 8a beschrieben. Das Wirbelsäulenimplantat 8a besteht aus mindestens einem Plattenverbund 801, 802, 803 und 804, einer Halterung 806, verschiedenen Verbindungselementen 8051, 8052, 8053 und 8054 und wenigstens einer Exzenterwelle 8055. Die Verbindungselemente 8051, 8052, 8053 und 8054 sind an den Lagerstellen 8065 mit der Halterung 806 verbunden. Des Weiteren kann die Halterung 806 geeignete Elemente 8062 zur Verankerung in der Deckplatte besitzen. Die Komponenten sind so angeordnet, dass bei Rotation der Exzenterwelle 8055 eine kombinierte Hub- und Translationsbewegung im Plattenverbund stattfindet, wie dies in Fig. 13 dargestellt ist. So ist es beispielsweise möglich, dass die Rotation 891-895 der exzentrischen Welle 8055 Peristaltikbewegungen zwischen den Platten 801 und 802 erzeugt, die in der Summe eine lineare Bewegung oder Translation 890 auf der Verzahnungsfläche des Implantats 8a ergeben. Diese mechanische Umsetzung kann dazu verwendet werden, dass ein fehlgestellter Wirbel reponiert und damit bezüglich seiner AP-Lage korrigiert wird. In Fig. 13 ist ein solcher Repositionsvorgang illustriert. Dazu wird das Implantat 8a in den Wirbelzwischenraum eingeführt 81. Das Ende mit der Halterung wird dabei an einer anterioren Wirbelkante 102 fixiert 82; hier beispielhaft mit einer Knochenschraube 9 gezeigt. Anschließend wird mit einem Instrument 7 eine rotatorische Bewegung auf die Exzenterwelle 8055 übertragen 83. Aufgrund der Rotation der Exzenterwelle 8055 übt das Wirbelsäulenimplantat eine Translationsbewegung 890 auf die Deckplatte des unteren Wirbels in AP-Richtung aus, wodurch der Wirbel positioniert bzw. seine Lage korrigiert werden kann 84.

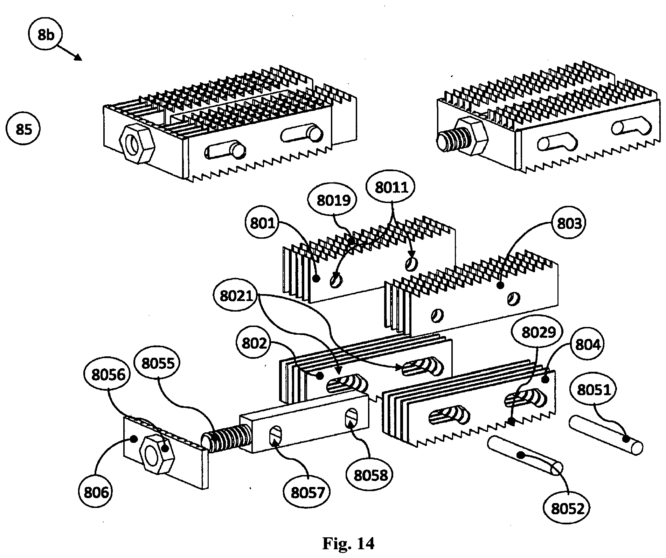

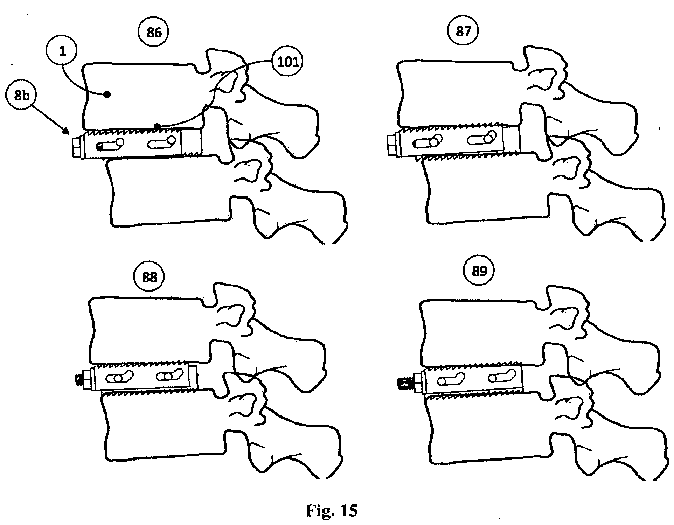

Eine modifizierte Ausführung eines Wirbelsäulenimplantats 8b mit Repositionsmechanismus ist in Fig. 14 dargestellt. Das Wirbelsäulenimplantat 8b besteht aus mindestens einem Plattenverbund 801, 802, 803 und 804, einer Halterung 806, verschiedenen Verbindungselementen 8051, 8052 und wenigstens einer Spindel 8055. Die Verbindungselemente 8051, 8052 sind an den Lagerstellen 8057 und 8058 mit der Spindel 8055 verbunden. Die Spindel 8055 ist zum Rahmen 806 mit einer Spindelmutter 8056 verbunden. Des Weiteren weisen die Platten zum Knochen hin Strukturen auf, die eine Verankerung der Deckplatten 101 in der Repositionsrichtung begünstigen 8019 und 8029. Die Spindel wird mit Hilfe einer Spindelmutter 8056 verschoben, dadurch wird ein Teil des Plattenverbundes 801 und 803 transportiert 85. Aufgrund des Verlaufs der Bohrungen und Schlitze 8011 und 8021 für die Lagerwellen 8051 und 8052 kann das Implantat zuerst in der Höhe distrahiert werden 87. Nach weiterem Verfahren der Spindel findet eine Translation statt 88, die für eine Translation und damit Reponierung 89 sorgt, wie dies in der Figur 15 dargestellt ist.

Die Fusion zweier Wirbel ist eine der am häufigsten durchgeführten Eingriffe der Wirbelsäulenchirurgie. Die vorgeschlagene Erfindung betrifft ein Wirbelsäulenimplantat, welches aus einer Vielzahl von parallel angeordneten Platten aufgebaut ist. Durch das gezielte Einbringen von Konturen in den Platten können biomechanisch vorteilhafte Funktionen und Einstellmöglichkeiten erzeugt werden, die auf andere Weise nicht oder nicht kostengünstig realisierbar sind. So kann sich das erfindungsgemäße Implantat durch eine Elastizität der Verankerungselemente an die knöchernen Deckplatten anpassen, um so für eine gleichmäßige Kraftverteilung zu sorgen und damit der Gefahr der Einsinterung bzw. des Deckplatteneinbruchs entgegenzuwirken. Durch den Plattenaufbau können Fertigungsverfahren verwendet werden, die die Herstellung von Haken-ähnlichen Hinterschnitt-Konturen ermöglichen und damit eine hervorragende Verankerung im Knochen erlauben ohne diesen zu schädigen. Außerdem können die Platten über einen Aktuator derart miteinander verbunden werden, dass sich diese in der Höhe und/oder im Winkel verstellen lassen und somit eine Aufrichtung und physiologische Einstellung des Bandscheibenfachs erlauben. Die Aufrichtung kann über die Länge des Implantats unterschiedlich sein, so dass auch eine Winkeleinstellung des Segments ermöglicht wird. Werden die Platten über eine Exzenterwelle miteinander verbunden, so kann die auf den Plattenverbund ausgeübte kombinierte Hub- und Translationsbewegung außerdem zur translatorischen Repositionierung zweier Wirbel verwendet werden.

## Patentansprüche

1. Medizinisches Implantat (2; 5a; 5b; 5c; 6d; 8a; 8b) zur Platzierung zwischen Knochen, insbesondere zwischen Wirbelkörper, wobei das Implantat eine Vielzahl von weitgehend parallel angeordneten, dünnen Platten (501, 502) umfasst, die über Verbindungselemente (23, 5051, 5052, 5053) miteinander verbunden sind, und wobei den Platten ein Aktuator (5052) zugeordnet ist, mit dem eine Position der Platten eingestellt werden kann, **dadurch gekennzeichnet, dass** der Aktuator (5000) mindestens eine Exzenterwelle (5051, 5052, 5053) enthält und die Exzenterwelle durch einen oder mehrere Exzenterzapfen mit gleichen oder verschiedenen Kurbelradien definiert ist, um eine Höheneinstellung für das Implantat zu ermöglichen.

2. Medizinisches Implantat gemäß Anspruch 1, **dadurch gekennzeichnet, dass** ein Abstand (22) zwischen den Platten (21) durch Ringe (5020) oder Scheiben, welche sich auf den Verbindungselementen (23, 5051, 5052, 5053) befinden, definiert ist.

3. Medizinisches Implantat gemäß einem der vorherigen Ansprüche, **dadurch gekennzeichnet, dass** der Abstand der Platten (21) durch versetzte Prägungen oder Ausformungen definiert ist.

4. Medizinisches Implantat gemäß einem der vorherigen Ansprüche, **dadurch gekennzeichnet, dass** das Verbindungselement (32) in Gestalt eines Streifens oder Platte ausgeführt ist, das Verbindungselement (32) über Schlitze (311) verfügt und der Abstand der Schlitze (311) den Abstand (22) der Platten (31) definiert.

5. Medizinisches Implantat gemäß einem der vorherigen Ansprüche, **dadurch gekennzeichnet, dass** die Platten (21, 31) über Aussparungen (413), Stege (423) oder Einschnitte (433) verfügen, die dazu geeignet sind die Steifigkeit des Implantats zu reduzieren.

6. Medizinisches Implantat gemäß einem der vorherigen Ansprüche, **dadurch gekennzeichnet, dass** die Platten (21, 31) an zu einer Deckfläche (101) orientierten Stirnseiten eine Verzahnung (212, 412, 422, 432) enthalten, deren Kontur so gestaltet ist, dass sich ein Hinterschnitt ergibt.

7. Medizinisches Implantat gemäß einem der vorherigen Ansprüche, **dadurch gekennzeichnet, dass** die Platten (21, 31) an zu einer Deckfläche (101) orientierten Stirnseiten eine Verzahnung (212, 412, 422, 432) enthalten, deren Zähne einzeln oder segmentweise über geeignete Aussparungen (413, 423, 433) in den Platten (21, 31) elastisch federnd bzw. nachgiebig gelagert sind.

8. Medizinisches Implantat gemäß einem der vorherigen Ansprüche, **dadurch gekennzeichnet, dass** die Aussparungen (213) semipermeabel sind oder eine Vielzahl von Bohrungen bzw. Poren enthält, die Aufgrund ihres Durchmessers zur Zelldifferentiation oder zum Aufbau eines osmotischen Drucks geeignet sind.

9. Medizinisches Implantat nach einem der Ansprüche 1 bis 8, **dadurch gekennzeichnet dass** der Aktuator (5000) eine Gewindespindel (5054, 5055, 6053), eine Zahnstange oder einen Rast- bzw. Klemmmechanismus umfasst.

10. Medizinisches Implantat nach einem der Ansprüche 1 bis 8, **dadurch gekennzeichnet, dass** der Aktuator (5000) einen elektrischen, elektromagnetischen oder piezoelektrischen Stellantrieb umfasst.

11. Medizinisches Implantat nach einem der der Ansprüche 1 bis 10, **dadurch gekennzeichnet dass** der Aktuator (5000) Verbindungselemente in Gestalt von Bolzen, Stiften, Achsen, Schrauben, Kulissensteinen oder Keilen beinhaltet, die innerhalb von Aussparungen (5011, 5012, 5013) oder Führungsnuten (6011, 6012, 6021, 6022) in den Platten (501, 502) verschiebbar sind.

12. Medizinisches Implantat gemäß einem der vorherigen Ansprüche, **dadurch gekennzeichnet, dass** die Platten (21, 32, 501, 502) über Verbindungselemente oder einen Stoff- oder Formschluss mit mindestens einer Aufnahme (5050) verbunden sind, und die Aufnahme (5050) mit mindestens einer Aussparung (571) versehen ist, die zur Aufnahme eines Knochenankers geeignet ist.

13. Medizinisches Implantat gemäß einem der vorherigen Ansprüche, **dadurch gekennzeichnet dass** die Kontur der Plattenaussparungen (5011-5014) und die Anordnung der Exzenternocken einer Exzenterwelle (8055) so aufeinander abgestimmt sind, dass eine Drehbewegung der Exzenterwelle (8055) in einer Hub- und Translationsbewegung (890) der Platten (801-804) resultiert, und zwar so dass die gleicher Drehrichtungsbewegung die Translationsbewegung (890) der jeweils distrahierenden Platten stets in dieselbe Richtung zeigt.

14. Medizinisches Implantat bestehend aus wenigstens zwei, jeweils aus einer Vielzahl von weitgehend parallel angeordneten, dünnen Platten gebildeten Plattenverbünden (803, 804) mit Aussparungen (8011, 8021), einem Aktuatormechanismus (8055, 8056) und Verbindungselementen (8051, 8052), **dadurch gekennzeichnet, dass** Konturen der Aussparungen (8011, 8021) derart ausgebildet sind, dass bei einer Verschiebung der mit dem Aktuatormechanismus (8055, 8056) verbundenen, als Achsen ausgebildeten Verbindungselemente (8051, 8052) ein Plattenverbund (803) gegenüber einem zweiten Plattenverbund (804) für eine Höhenverstellung des Implantats distrahiert wird.

15. Medizinisches Implantat nach Anspruch 14, **dadurch gekennzeichnet, dass** die Kontur der Aussparungen (8011, 8021) derart ausgebildet ist, dass bei einer Verschiebung der mit dem Aktuator verbundenen, als Achsen ausgebildeten Verbindungselemente (8051, 8052) für den Plattenverbund (803) gegenüber dem zweiten Plattenverbund (804) nach der Distraktion bei weiterer Verschiebung der Achsen eine in AP-Richtung wirkende Translationsbewegung zwischen den Plattenverbünden (803, 804) herbeigeführt wird.

## Claims

1. Medical implant (2; 5a; 5b; 5c; 6d; 8a; 8b) for placing between bones, in particular between vertebral bodies, wherein the implant includes a multiplicity of thin plates (501, 502) in a largely parallel arrangement and connected to one another via connecting elements (23, 5051, 5052, 5053), and wherein the plates are assigned an actuator (5052), by which a position of the plates may be set, **characterised in that** the actuator (5000) includes at least one eccentric shaft (5051, 5052, 5053) and the eccentric shaft is defined by one or more eccentric pins with the same or different crank radii, in order to facilitate height setting for the implant.

2. Medical implant according to claim 1, **characterised in that** the spacing (22) between the plates (21) is defined by rings (5020) or discs, which are provided on the connecting elements (23, 5051, 5052, 5053).

3. Medical implant according to any of the preceding claims, **characterised in that** the spacing (22) of the plates (21) is defined by embossing or mouldings.

4. Medical implant according to any of the preceding claims, **characterised in that** the connecting element (32) is in the form of a strip or plate, the connecting element (32) has slits (311), and the spacing of the slits (311) defines the spacing (22) of the plates (31).

5. Medical implant according to any of the preceding claims, **characterised in that** the plates (21, 31) have recesses (413), webs (423) or notches (433) which are suitable for reducing the rigidity of the implant.

6. Medical implant according to any of the preceding claims, **characterised in that** the plates (21, 31) contain, on end faces oriented towards a top surface (101), a tooth system (212, 412, 422, 432) which has a contour so shaped as to produce an undercut.

7. Medical implant according to any of the preceding claims, **characterised in that** the plates (21, 31) contain, on end faces oriented towards a top surface (101), a tooth system (212, 412, 422, 432) with teeth which are mounted, elastically sprung or flexible, individually or in segments, via suitable recesses (413, 423, 433) in the plates (21, 31).

8. Medical implant according to any of the preceding claims, **characterised in that** the recesses (213) are semi-permeable or contain a multiplicity of drilled holes or pores which, on account of their diameter, are suitable for cell differentiation or for the build-up of an osmotic pressure.

9. Medical implant according to any of claims 1 to 8, **characterised in that** the actuator (5000) includes a threaded spindle (5054, 5055, 6053), a gear rack or a latching or clamping mechanism.

10. Medical implant according to any of claims 1 to 8, **characterised in that** the actuator (5000) includes an electrical, electromagnetic or piezoelectric actuator drive.

11. Medical implant according to any of claims 1 to 10, **characterised in that** the actuator (5000) contains connecting elements in the form of bolts, pins, shafts, screws, slide blocks or wedges, which are movable within recesses (5011, 5012, 5013) or guide slots (6011, 6012, 6021, 6022) in the plates (501, 502).

12. Medical implant according to any of the preceding claims, **characterised in that** the plates (21, 32, 501, 502) are connected via connecting elements or an adhesive bond or by form closure to at least one seat (5050), and the seat (5050) is provided with at least one recess (571) which is suitable for accommodating a bone anchor.

13. Medical implant according to any of the preceding claims, **characterised in that** the contour of the plate recesses (5011-5014) and the arrangement of the eccentric cams of an eccentric shaft (8055) are so coordinated that a rotary movement of the eccentric shaft (8055) results in a stroke and translatory movement (890) of the plates (801-804), namely so that the same rotary directional motion of the translatory movement (890) of the respectively distracted plates is always in the same direction.

14. Medical implant comprised of at least two plate groups (803, 804), each formed from a multiplicity of largely parallel thin plates, with recesses (8011, 8021), an actuator mechanism (8055, 8056) and connecting elements (8051, 8052), **characterised in that** contours of the recesses (8011, 8021) are so designed that, during movement of the connecting elements (8051, 8052) in the form of shafts and connected to the actuator mechanism (8055, 8056), one plate group (803) is distracted relative to the second plate group (804) for height adjustment of the implant.

15. Medical implant according to claim 14, **characterised in that** the contour of the recesses (8011, 8021) is so designed that, during movement of the connecting elements (8051, 8052) in the form of shafts for the plate group (803) relative to the second plate group (804), after the distraction, with further movement of the shafts, a translatory movement in the AP direction is generated between the plate group (803, 804).

## Revendications

1. Implant médical (2 ; 5a ; 5b ; 5c ; 6d ; 8a ; 8b) destiné à être placé entre des os, en particulier entre des vertèbres, dans lequel l'implant comprend une pluralité de plaques (501, 502) fines disposées de manière largement parallèle, lesquelles sont reliées les unes aux autres par l'intermédiaire d'éléments de liaison (23, 5051, 5052, 5053), et dans lequel un actionneur (5052) est associé aux plaques, avec lequel une position des plaques peut être réglée, **caractérisé en ce que** l'actionneur (5000) contient au moins un arbre excentrique (5051, 5052, 5053) et l'arbre excentrique est défini par un ou plusieurs tourillons excentriques avec des rayons de manivelle identiques ou différents, afin de permettre un réglage en hauteur de l'implant.

2. Implant médical selon la revendication 1, **caractérisé en ce qu'**un espacement (22) entre les plaques (21) est défini par des anneaux (5020) ou des disques, qui se trouvent sur les éléments de liaison (23, 5051, 5052, 5053).

3. Implant médical selon l'une quelconque des revendications précédentes, **caractérisé en ce que** l'espacement des plaques (21) est défini par des empreintes ou déformations décalées.

4. Implant médical selon l'une quelconque des revendications précédentes, **caractérisé en ce que** l'élément de liaison (32) est réalisé sous la forme d'une bande ou d'une plaque, l'élément de liaison (32) dispose d'entailles (311), et l'espacement des entailles (311) définit l'espacement (22) des plaques (31).

5. Implant médical selon l'une quelconque des revendications précédentes, **caractérisé en ce que** les plaques (21, 31) disposent d'évidements (413), d'entretoises (423) ou d'incisions (433), qui sont adaptés pour réduire la rigidité de l'implant.

6. Implant médical selon l'une quelconque des revendications précédentes, **caractérisé en ce que** les plaques (21, 31) contiennent, au niveau de côtés frontaux orientés en direction d'une surface de recouvrement (101), une denture (212, 412, 422, 432), dont le contour est configuré de manière à faire apparaître une contre-dépouille.

7. Implant médical selon l'une quelconque des revendications précédentes, **caractérisé en ce que** les plaques (21, 31) contiennent, au niveau de côtés frontaux orientés en direction d'une surface de recouvrement (101), une denture (212, 412, 422, 432), dont les dents sont montées sur ressorts de manière élastique ou de manière souple individuellement ou par segments sur des évidements (413, 423, 433) adaptés dans les plaques (21, 31).

8. Implant médical selon l'une quelconque des revendications précédentes, **caractérisé en ce que** les évidements (213) sont semi-perméables ou contiennent une pluralité d'alésages ou de pores, qui sont adaptés, du fait de leur diamètre, pour la différenciation de cellules ou pour établir une pression osmotique.

9. Implant médical selon l'une quelconque des revendications 1 à 8, **caractérisé en ce que** l'actionneur (5000) comprend une broche filetée (5054, 5055, 6053), une tige dentée ou un mécanisme d'enclenchement ou de serrage.

10. Implant médical selon l'une quelconque des revendications 1 à 8, **caractérisé en ce que** l'actionneur (5000) comprend un entraînement de réglage électrique, électromagnétique ou piézoélectrique.

11. Implant médical selon l'une quelconque des revendications 1 à 10, **caractérisé en ce que** l'actionneur (5000) renferme des éléments de liaison sous la forme de boulons, de tiges, d'axes, de vis, de coulisseaux ou de cales, qui peuvent être coulissés à l'intérieur d'évidements (5011, 5012, 5013) ou de rainures de guidage (6011, 6012, 6021, 6022) dans les plaques (501, 502).

12. Implant médical selon l'une quelconque des revendications précédentes, **caractérisé en ce que** les plaques (21, 32, 501, 502) sont reliées à au moins un logement (5050) par l'intermédiaire d'éléments de liaison ou d'une liaison de matière ou à complémentarité de forme, et le logement (5050) est pourvu d'au moins un évidement (571), qui est adapté pour recevoir un système d'ancrage d'os.

13. Implant médical selon l'une quelconque des revendications précédentes, **caractérisé en ce que** le contour des évidements de plaque (5011 - 5014) et l'agencement des cames excentriques d'un arbre excentrique (8055) sont adaptés les uns aux autres de sorte qu'un déplacement par rotation de l'arbre excentrique (8055) donne lieu à un déplacement de levage et par translation (890) des plaques (801 - 804), et ce, de sorte que le déplacement dans un sens de rotation identique montre le déplacement par translation (890) des plaques se séparant respectivement toujours dans la même direction.

14. Implant médical constitué d'au moins deux, respectivement d'une pluralité de composites de plaque (803, 804) disposés de manière largement parallèle, formant des plaques fines, pourvus d'évidements (8011, 8021), d'un mécanisme actionneur (8055, 8056) et d'éléments de liaison (8051, 8052), **caractérisé en ce que** des contours des évidements (8011, 8021) sont réalisés de telle manière que lors d'un coulissement des éléments de liaison (8051, 8052) reliés au mécanisme actionneur (8055, 8056), réalisés sous la forme d'axes, un composite de plaque (803) est séparé par rapport à un deuxième composite de plaque (804) en vue d'un ajustement en hauteur de l'implant.

15. Implant médical selon la revendication 14, **caractérisé en ce que** le contour des évidements (8011, 8021) est réalisé de telle manière que dans le cas d'un coulissement des éléments de liaison (8051, 8052) reliés à l'actionneur, réalisés sous la forme d'axes, pour le composite de plaque (803) par rapport au deuxième composite de plaque (804), un déplacement par translation agissant dans la direction AP est entraîné entre les composites de plaque (803, 804) après la séparation dans le cas d'un autre coulissement des axes.
